(19) Europäisches Patentamt

European Patent Office

Office européen des brevets

(11) Publication number: 0 209 882
A2

# (12) EUROPEAN PATENT APPLICATION

(21) Application number: 86110016.2

(22) Date of filing: 21.07.86

(51) Int. Cl.⁴: **C12P 19/04** , C12N 5/00 ,
D01F 2/00 ,
//(C12P19/04,C12R1:91)

(30) Priority: 26.07.85 US 759548

(43) Date of publication of application:
28.01.87 Bulletin 87/05

(84) Designated Contracting States:
BE CH DE FR GB IT LI

(71) Applicant: **Research Corporation
Suite 853, 25 Broadway
New York New York 10004(US)**

(72) Inventor: **Goodin, Joe Ray
4014 69th Street
Lubbock Texas(US)**
Inventor: **Berlin, Jerry D.
7922 Joliet Street
Lubbock Texas(US)**
Inventor: **Trolinder, Norma L.
4015 89th Street
Lubbock Texas(US)**

(74) Representative: **Patentanwälte Grünecker,
Kinkeldey, Stockmair & Partner
Maximilianstrasse 58
D-8000 München 22(DE)**

(54) In vitro production of cotton fibers.

(57) This invention relates to a method for the in vitro
production of cotton fibers comprising:

providing a cell suspension of a strain of Gossypium

culturing said cell suspension in a liquid basal salts
medium supplemented with a carbon source under
conditions of time and temperature sufficient to pro-
mote cotton fiber production.

# FIG.1

1a

## IN VITRO PRODUCTION OF COTTON FIBERS

This invention relates to a process for the production of plant products in cell culture. More specifically, the invention relates to the production of cotton fibers in vitro by culturing cells of various strains of Gossypium.

Cotton is an economically important crop in that it supplies five major products: lint, oil, seed hulls, meal, and linters, the lint being the most important, providing much of the textile industry with high quality fiber. The oil is employed in shortening, cooking oil, mayonnaise, etc. Cotton seed hulls are useful as cattle feed or as a soil mulch. Meal from glandless seed or gossypol-extracted seed is used as a protein supplement for livestock. Linters are used as a source of cellulose for the plastics industry and as batting for cushions.

This invention relates to a process for generating cotton fibers in cell culture, thus providing an alternative source for fiber production which under controlled conditions can result in a product which is continuously available, uniform, and displaying particularly desired properties. Further advantages include the elimination of disease resistance and other agronomic considerations as criteria for variety selection.

Investigations concerning the morphogenesis of plant tissue in culture date back at least to the 1950's (Skoog, F. and Miller C.O., SympSoc. Exp. Biol., 11:118 (1957)) and have continued apace to date. Several monographs provide extensive reviews of the field and contain compilations of numbers of species which will undergo plant regeneration in culture (See for example, Murashigi T., In: "Propagation of Higher Plants through Tissue Culture," T.A. Thorpe, Ed., p. 15, Univ. Calgary Press, Calgary (1978); Vasil, I,K, et al. Adv. Gent. 20:127 - (1979) and Evans. D. A., et al. In: "Plant Tissue Culture: Methods and Applications in Agriculture: T.A. Thorpe, Ed. pg. 45, Academic Press, New York (1981)).

The term "plant tissue culture" as used herein is taken in its broadest meaning to refer to the cultivation, in vitro, of any plant parts, whether a single cell, a tissue or an organ, under aseptic conditions. More restrictive terms relating to plant tissue culture technology include: "callus culture" which means the culture of cell masses on agar medium and produced from an explant of a seedling or other plant source; "cell suspension culture" which means the culture of cells in liquid media in vessels which are usually aerated by agitation; "organ culture" which means, the aseptic culture on nutrient media of embryos, anthers (including microspores), ovules, roots, shoots, or other plants organs; "meristem culture and morphogenesis" which means the aseptic culture of shoot meristems or other explant tissue on nutrient media for the purpose of growing complete plants, and "protoplast culture" which means the aseptic isolation and culture of plant protoplasts from cultured cells or plant tissue.

Six types of plant tissue culture have been described for cotton. These include: callus, ovule-embryo, shoot tip, anther, protoplast, and cell suspension culture including somatic embryogenesis. For a review of each of these categories, see: H.J. Price and R.H. Smith Cotton IN: Handbook of Plant Cell Culture, P.V. Ammirato et al. , Eds, Macmillan Publishing Co., Vol. 3, Chapter 18, 487-510 (1984).

For purposes of this invention, callus culture, cell suspension culture and ovule-embryo culture are the most important. Callus culture has been described for at least seven species of Gossypium including: G. hirsutum, G.asboreum, G. barbadense, G. anomalum, G. armourianum, G. klotzschianum, and G. raimondii. The subject invention contemplates the use of any Gossypium species or strains thereof as starting material for the production of cotton fibers in vitro.

The production of cotton fibers in vitro has been reported but such production has been restricted to ovule culture systems. (See: Beasley, C.A. & I.P. Ting, Amer. J.Bot. 60: 130-139 (1973); Beasley, C.A. & I.P. Ting, Amer. J. Bot. 61: 188-194 (1974); Benedict, C.R. et al. Agronomy Abst-1980 Pg 76; Dhindsa, R.S. et al. Planta 130 (2): 197-201 (1976)). As distinguished from the above reports this invention provides a method for in vitro cotton fiber production from a cell suspension derived from callus.

This invention relates to a method for the in vitro production of cotton fibers comprising:

providing a cell suspension of a strain of Gossypium

culturing said cell suspension in a liquid basal salts medium supplemented with a carbon source under conditions of time and temperature sufficient to promote cotton fiber production.

In a further embodiment this invention relates to a method for the in vitro production of cotton comprising:

providing a cell suspension of a strain of Gossypium ;

culturing said cell suspension in liquid medium comprising basal salts, a carbon source, a growth regulator and ovule conditioned medium.

Figure 1 illustrates the in vitro produced cotton fibers. Suspended cotton cells initially assume a spherical shape (A), then begin to elongate (B) to become increasingly longer cotton fibers (C, D and E). These cotton fibers were prepared during the elongation phase and, therefore, lack a secondary cell wall. Dark-field light microscopy of a whole mount. The magnification is approximately 100 X.

Figure 2 shows a suspended cotton cell that has elongated at two sites, therefore, in vitro produced cotton fibers may elongate from either one or both ends of the cell. Dark-field light microscopy of a whole mount. The magnification is approximately 100 X.

Figure 3 shows an in vitro produced cotton fiber. Bright-field light micrograph of a whole mount. The magnification is approximately 1230 X.

Figure 4 shows the cotton fiber in Figure 3 by polarized microscopy to have a secondary cell wall. The magnification is approximately 1230 X.

Detailed Description of the Invention

It has been discovered that a number of strains of Gossypium are capable of producing cotton fibers in vitro when cells derived from callus are cultured on appropriately designed media. Strains which have been observed to produced fibers when cultured according to this invention include:

T25 ( G. hirsutum, Texas Collection)

P784 (G. hirsutum, Paymaster)

RQSX1-1 (G. hirsutum X G. barbadense hybrid, Texas Collection)

C312 (G. hirsutum, Coker)

ST213 (G. hirsutum, Stoneville)

SJ1, SJ2, SJ5 (G. hirsutum, Acala type)

Fiber Identification

Fibers were identified by observation with a Zeiss Stereomicroscope, a Zeiss Ultraphot Reichert Zetopan polarizing microscope, and a Zeiss Inverted Phase-Contrast Microscope. A stage micrometer was used for calibrating magnification prior to photography. Elongated cells in excess of 500 um in length were considered to be potential fiber cells. Birefringence, as observed with polarizing microscopy, was evidence for the presence of secondary cell walls.

Criteria for Evaluation of Fiber Production

From a population of liquid suspension cells, the percentage of cells elongating (length exceeding 2 mm) was determined. Number of days to induction of elongation, number of days to onset of secondary cell wall synthesis, and number of days to maturity are factors in fiber production.

Using these criteria, various environmental, nutritional, and hormonal parameters, as well as source material, can be evaluated.

Any basic salts medium which has been shown to be useful for callus culture of cotton would be useful in practicing this invention. Particularly preferred basic salts media include those of Beasely-Ting (BT) (Beasley, C.A. and I.P. Ting, Amer. J. Botany 60: 130-139 (1973) and Murashige-Skoog (MS) (Murashige, T. and F. Skoog, Physiol. Plant 15: 473 (1962). The MS basal medium was made minus $NH_4NO_3$, and $KNO_3$ was added as nitrogen source as described herein.

It was found to be necessary to supplement the above-described basal salts media with various additives. Useful additives appear in Table 1. In addition various nucleic acid and protein synthesis inhibitors may also be useful.

## TABLE 1

### Media and Additives Useful for in vitro Production of Cotton Fibers

auxins:

| | | |
|---|---|---|
| IAA | = | indole acetic acid; |
| IBA | = | indole butyric acid; |
| 2,4-D | = | 2,4-dichlorophenoxyacetic acid; |
| NAA | = | naphthaleneacetic acid; |
| pCPA | = | para-chlorophenoxyacetic acid; |
| NOA | = | B-napthoxyacetic acid; |
| BTOA | = | 2-benzothiazole acetic acid; |
| PIC | = | picloram; |
| 2,4,5-T | = | 2,4,5,-trichlorophenoxyacetic acid; |

cytokinins:

| | | |
|---|---|---|
| KIN | = | kinetin; |
| 6BA | = | 6 benzyladenine (benzylaminopurine) |
| 2iP | = | 2 isopentenyl adenine; |
| ZEA | = | zeatin; |

other growth regulators:

| | | |
|---|---|---|
| ADE | = | adenine; |
| CW | = | coconut water; |
| CH | = | casein hydrolysate; |
| ABA | = | abscisic acid; |
| GA | = | gibberellic acid and other gibberellins |

various phenolic compounds

basal medium

| | | |
|---|---|---|
| MS | = | Murashige and Skoog |
| B5 | = | Gamborg |
| SH | = | Schenk and Hildebrandt |
| W | = | White |
| LS | = | Linsmaier and Skoog |
| BT | = | Beasley and Ting |

Particularly effective additives include IAA at a concentration from about 0 to about 10 mg/l most preferably from about 0 to about 3 mg/l; kinetin at a concentration from about 0 to about 1 mg/l, most preferably from about 0 to about 0.1 mg/l; gibberellic acid at a concentration of about 0 to about 10 mg/l most preferably from about 1 mg/l to about 3 mg/l; polyethylene glycol 8000 (PEG) at a concentration of about 0 to about 300 g/l, preferably from about 100 g/l to about 200 g/l; UDP glucose at a concentration of about 0 to about $10^{-5}$M prefer-

ably about 0 to about $6 \times 10^{-5}$M. It was also discovered that "ovule-conditioned" medium was a particularly useful supplement. "Ovule-conditioned" medium supplement was prepared by

1) adding 5, two-days post-anthesis greenhouse-grown ovules/20 mls cell suspension or

2) adding supernatant prepared from 16, 2 day-old, post-anthesis greenhouse-grown cotton ovules which had been ground with a tissue grinder and pelleted at 15,000 rpm for 15 minutes.

Nitrogen in the form of $KNO_3$ was present at a concentration of from about 1.9 g/l to about 5.7 g/l, preferably from about 3.8 g/l to about 5.7 g/l and a carbon source such as glucose or sucrose was added at a concentration of from about 20 g/l to about 40 g/l preferably from about 22 g/l to about 30 g/l.

In one embodiment of the invention cotton fiber production was observed after cells were cultured in a medium comprising basic MS salts supplemented with 30 g/l glucose. Even though fiber production is rare in this medium, a few fiber cells do occur.

In another embodiment improved fiber production was observed in a medium comprised of basic MS salts supplemented with 1.9 g/l $KNO_3$ and 2 mg/l $GA_3$. This medium caused cotton suspension cells to develop more fibers than in the medium described above.

The most preferred composition is a medium containing BT salts supplemented with 2 mg/l $GA_3$ and cotton ovules as described earlier This is the so-called "ovule-conditioned medium".

All the above experiments were performed with Gossypium hirsutum variety P784, but evidence indicates that even better results are obtained with the Acala variety SJ1 using the same media.

As with any in vitro system, the temperature of incubation is an important parameter. Fiber production has been observed in cultures growing at temperatures from about 25°C to 34°C, preferably from about 29°C to about 34°C.

Although without limiting the subject invention, the following example further illustrates specific embodiment thereof.

EXAMPLE I

This example demonstrates the production of cotton fibers by cotton cells in culture.

Seeds were germinated in August of 1984 of variety Paymaster 784, and hypocotyl sections were placed on a MS basal salt medium supplemented with 0.1 mg/l 2,4-D and 0.1 mg/l kinetin. Callus from these tissues were grown on an agar medium at 29°C ± 2°C in a room with 90 mE/m²/sec light for 16 hours and 8 hours darkness. The tissues were routinely subcultured onto the same medium at intervals of 30-45 days. On April 25, 1985, callus was transferred to a BT medium with conditioned ovules as described previously + 2 mg/l $GA_3$ in liquid suspension. Cells were cultured in darkness at 29°C ± 2°C, , and rotated on a shaker at 120 rpm.

The first fibers were detected on May 8, 1985, and the first secondary cell walls were detected on these fibers on May 21, 1985.

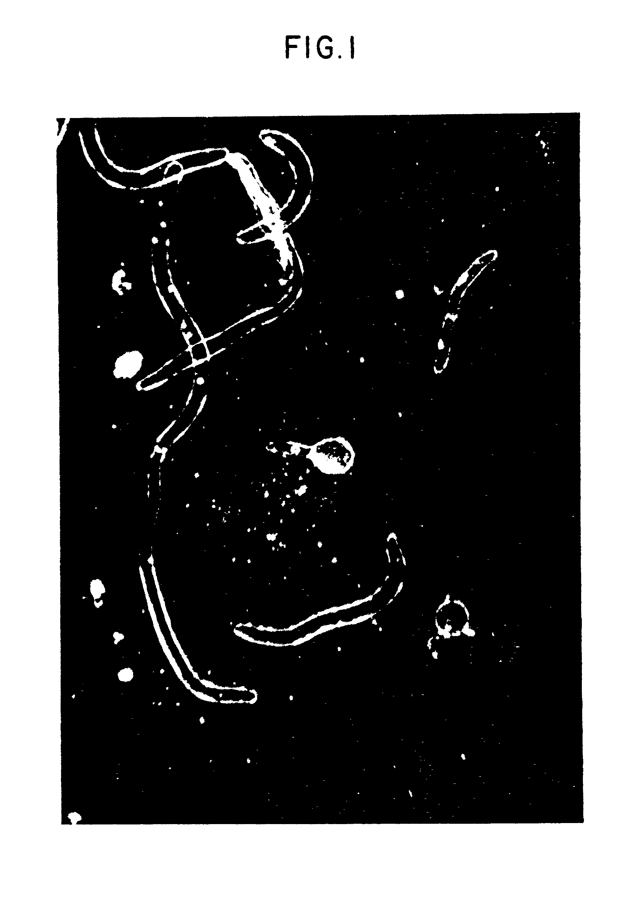

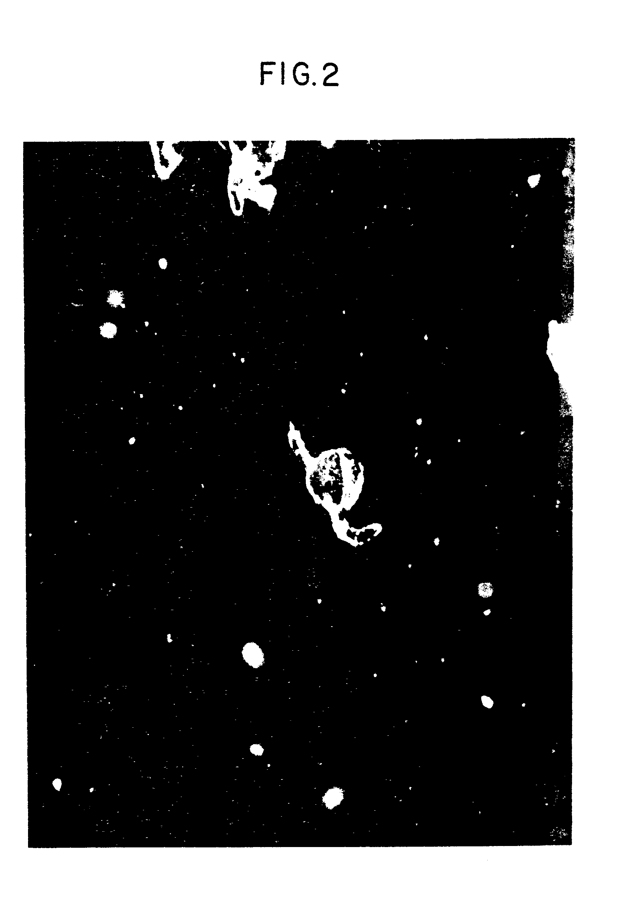

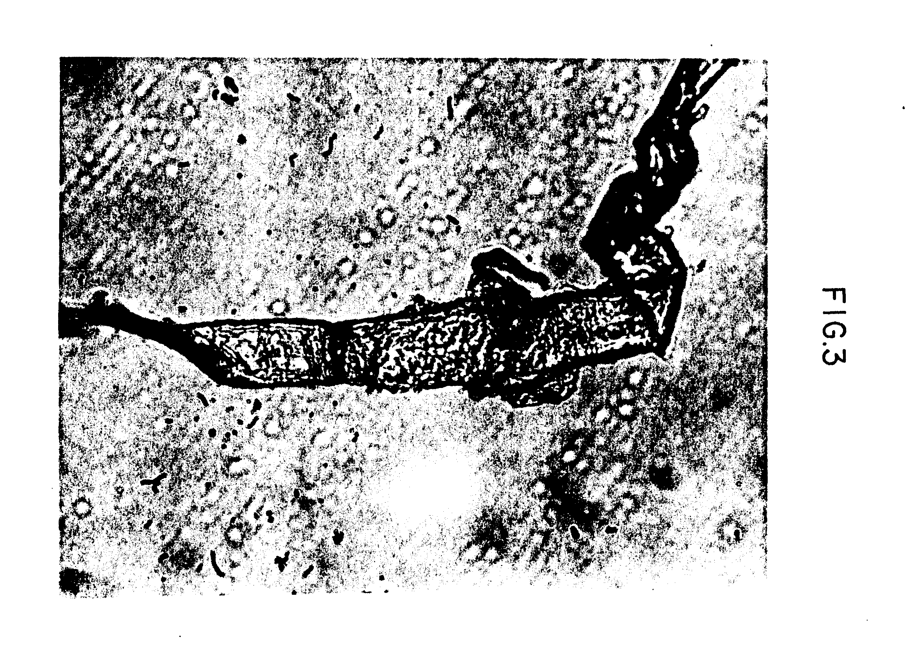

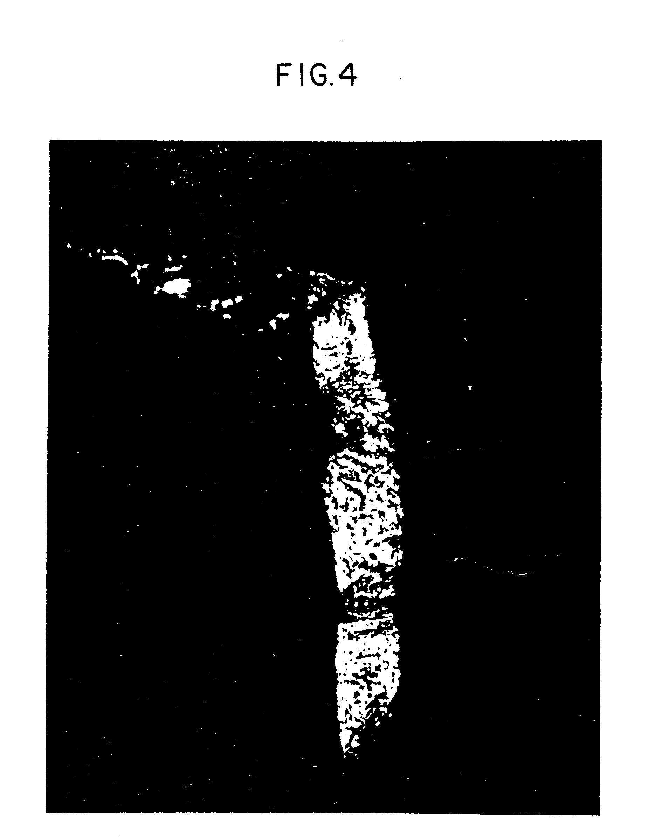

The fibers produced by this method are depicted in Figures 1-4.

## Claims

1. A method for the in vitro production of cotton fibers comprising:

providing a cell suspension of a strain of Gossypium

culturing said cell suspension in a liquid basal salts medium supplemented with a carbon source under conditions of time and temperature sufficient to promote cotton fiber production.

2. The method according to Claim 1 wherein the Gossypium strain is selected from the group consisting of G. hirsutum, G. arboreum , G. barbadense, G. anomalum , G. armourianum, G. klotzchianum and G. raimondii.

3. The method according to Claim 2 wherein said G.hirsutum strain is selected from the group consisting of T25, P784, RQSX-1, C312, ST213, SJ1, SJ2 and SJ5.

4. The method according to Claim 1 wherein said basal salts medium is selected from the group consisting of Murashige and Skoog, Gamborg, Schenk and Hildebrandt, White, Linsmaier and Skoog, and Beasley and Ting.

5. The method according to Claim 1 wherein said carbon source is glucose at a concentration of about 20 to about 40 g/l.

6. The method according to Claim 1 wherein the cell suspension in provided by:

forming a callus by placing explants of tissue selected from the group consisting of cotyledon, hypocotyl, stems, leaf, shoot apex, root, ovule, flower petals, and petioles on a solid basal salts medium containing an auxin and a cytokinin and culturing said tissue at about 29°C under about 16 hours of light at about 90 mE/m²/sec and about 8 hours of darkness.

subculturing the callus at intervals of 30-45 days. forming a cell suspension by transferring the callus into a liquid medium.

7. The method according to Claim 6 wherein said tissue explant is hypocotyl.

8. The method according to Claim 6 wherein said auxin is 2,4-D and said cytokinin is kinetin.

9. The method according to Claim 1 wherein said liquid medium is further supplemented with a nitrogen source and a growth regulator.

10. The method according to Claim 9 wherein said nitrogen source is KNO$_3$ and said regulator is gibberellic acid.

11. The method according to claims 9 or 10 wherein said medium is further supplemented with ovule conditioned medium.

12. The method according to any of claims 1-11 wherein said culturing step is conducted at about 29°C in the dark.

13. Cotton fibers when produced by the method of any of the claims 1-12.

## FIG.1

## FIG.2

## FIG.3

# FIG.4